(19) Europäisches Patentamt European Patent Office Office européen des brevets

(11) **EP 3 892 265 A1**

(12) **EUROPEAN PATENT APPLICATION**
published in accordance with Art. 153(4) EPC

(43) Date of publication:
**13.10.2021 Bulletin 2021/41**

(21) Application number: **19892470.6**

(22) Date of filing: **02.12.2019**

(51) Int Cl.:
*A61K 31/121* (2006.01)   *A61P 9/12* (2006.01)
*A61P 43/00* (2006.01)   *A23L 33/105* (2016.01)
*A23L 2/52* (2006.01)

(86) International application number:
**PCT/JP2019/046983**

(87) International publication number:
**WO 2020/116382 (11.06.2020 Gazette 2020/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.12.2018 JP 2018229131**

(71) Applicant: **Suntory Holdings Limited**
**Osaka 530-8203 (JP)**

(72) Inventors:
• **FUKIZAWA, Shinya**
  **Soraku-gun, Kyoto 619-0284 (JP)**
• **TAKAGI, Risa**
  **Soraku-gun, Kyoto 619-0284 (JP)**

(74) Representative: **Vossius & Partner**
**Patentanwälte Rechtsanwälte mbB**
**Siebertstraße 3**
**81675 München (DE)**

(54) **COMPOSITION FOR SUPPRESSING BLOOD PRESSURE ELEVATION AND METHOD FOR SUPPRESSING BLOOD PRESSURE ELEVATION**

(57)    The present invention aims to provide a novel composition for suppressing blood pressure elevation, a novel method for suppressing blood pressure elevation, and the like, which are capable of suppressing blood pressure elevation. The present invention relates to a composition for suppressing blood pressure elevation and the like which contain xanthohumol as an active ingredient.

EP 3 892 265 A1

**Description**

TECHNICAL FIELD

The present invention relates to a composition for suppressing blood pressure elevation and a method for suppressing blood pressure elevation.

BACKGROUND ART

High blood pressure (also referred to as hypertension) is a condition in which the blood pressure is abnormally high, and is one of lifestyle-related diseases with highest frequency of hospital visits. According to "Guidelines for the Management of Hypertension" by the Japanese Society of Hypertension, the blood pressure in adults is classified into the following groups: optimal blood pressure, normal blood pressure, high-normal blood pressure, grade I hypertension, grade II hypertension, grade III hypertension, and systolic blood pressure. When the systolic blood pressure is maintained at 140 mmHg or higher or the diastolic blood pressure is maintained at 90 mmHg or higher (i.e., other than optimal blood pressure, normal blood pressure, and high-normal blood pressure in the above classification), such a condition is defined as high blood pressure.

Complications of high blood pressure include cerebrovascular disease, heart disease, kidney disease, and vascular disease, for example. It is important to maintain normal blood pressure to prevent the onset of these diseases.

The renin-angiotensin system is involved in blood pressure regulation effect and maintenance of homeostasis of body fluids and electrolytes in living bodies, and is also known to be deeply involved not only in blood pressure elevation but also in onset of hypertension. In the renin-angiotensin system, angiotensin II, which constricts peripheral capillaries and stimulates sympathetic nerves and the adrenal gland to promote catecholamine release, elevates blood pressure. Angiotensin-converting enzyme (ACE) present in vascular endothelial cells and elsewhere plays an important role in angiotensin II production. Thus, pharmaceutical products that inhibit the activity of angiotensin-converting enzyme (i.e., ACE inhibitory effect), such as captopril and enalapril, have been used as antihypertensive agents.

Food protein-derived linear peptides are also known to have an ACE inhibitory effect. Many reports have been made on antihypertensive effects associated therewith, and foods for specified health uses containing, for example, sesame peptide (its indicator component is a tripeptide consisting of leucine, valine, and tyrosine (LVY)) as a functional substance have been made available.

Among food protein-derived peptides, a "dipeptide" consisting of two amino acids bound together has also been attracting attention as a functional component. Dipeptides can add physical and/or chemical properties and new functions not found in simple amino acids. Regarding the ACE inhibitory effect, for example, specific linear dipeptides derived from fish and shellfish have been reported as being useful.

While various pharmaceutical products have been used for antihypertensive effects, these products may cause side effects. In this regard, food protein-derived linear peptides are considered to be useful. However, various peptidases such as carboxypeptidase and aminopeptidase that are secreted into the gastrointestinal tract may act to decompose linear dipeptides into free amino acids (Non-Patent Literature 1), possibly reducing the adsorption of the linear dipeptides into the body. In view of the above, there has been a demand for a component and a composition that have fewer side effects, that are not decomposed by peptidases in the gastrointestinal tract, and that have an ACE inhibitory effect.

Non-Patent Literature 2 enumerates differences in the intensity of the ACE inhibitory effect by various naturally occurring flavonoids. According to the literature, among compounds classified as flavonoids, some show a strong ACE inhibitory effect and some show no ACE inhibitory effect, depending on their types. Thus, it is difficult to infer which specific flavonoids have an ACE inhibitory effect and how the intensity of the effect is different.

Vascular endothelial cells make up the lining of blood vessels, and are known to have a function to produce physiologically active substances such as nitric oxide (NO). Nitric oxide is one of vascular endothelium-derived relaxing factors, and is produced as a by-product of a reaction *in vivo* in which L-arginine is converted into L-citrulline by NO synthase (NOS). Promoting nitric oxide production from vascular endothelial cells is effective in relaxing and widening blood vessels and thus suppressing blood pressure elevation. For example, according to Non-Patent Literature 3, an antihypertensive effect was observed in spontaneously hypertensive rats (SHRs) orally ingested with hesperetin having an effect of promoting NO production, and the effect was attenuated when hesperetin was used in combination with L-NAME that is a NO synthase (NOS) inhibitor. In other words, it has been proven that NO produced from vascular endothelial cells contributes to suppressing blood pressure elevation via vascular relaxation.

As described above, blood pressure is controlled not only by the renin-angiotensin system but also by multiple regulatory mechanisms, such as vascular relaxation via NO production from vascular endothelial cells. There has been a demand for a highly safe component that acts on not only a single mechanism of action but also on multiple targets.

Xanthohumol is a compound present in a hop (scientific name: *Humulus lupulus*) cone, a plant of the Cannabis family, which is used as an ingredient of beer. Xanthohumol is known to have various physiological activities such as cancer cell growth inhibitory effect, antioxidant effect, effect of inhibiting bone breakdown, and antibacterial effect.

Attempts have been made to increase the amount of xanthohumol in various beverages in anticipation of maintenance and improvement of health by the above-described physiological effects of xanthohumol (Patent Literature 1 and Patent Literature 2).

Patent Literature 3 discloses an angiotensin I-converting enzyme inhibitory effect (ACE inhibitory effect) of hop leaf extracts instead of hop cones.

CITATION LIST

- Patent Literature

Patent Literature 1: JP 2002-345433 A
Patent Literature 2: JP 2003-310240 A
Patent Literature 3: JP 2012-153659 A

- Non-Patent Literature

Non-Patent Literature 1: J Biochem., Aug; 94(2): 619-22, 2008
Non-Patent Literature 2: PloS.ONE 7(11): e49493
Non-Patent Literature 3: J Nutr Sci Vitaminol, 54, 95-98

SUMMARY OF INVENTION

- Technical Problem

Patent Literature 3 describes an ACE inhibitory effect of hop leaf extracts, but hop leaf extracts contain various components, and which component contributes to the ACE inhibitory effect is unknown.

The present invention aims to provide a novel composition for suppressing blood pressure elevation, a novel method for suppressing blood pressure elevation, and the like, which are capable of suppressing blood pressure elevation.

- Solution to Problem

As a result of intensive studies to solve the above problems, the present inventors found that xanthohumol present in hop cones has an antihypertensive effect, and thus achieved the present invention.

Specifically, the present invention relates to, but is not limited to, a composition for suppressing blood pressure elevation, a method for suppressing blood pressure elevation, and the like described below.

(1) A composition for suppressing blood pressure elevation, the composition containing xanthohumol as an active ingredient.
(2) The composition for suppressing blood pressure elevation according to (1) above, wherein the composition suppresses blood pressure elevation by inhibiting angiotensin-converting enzyme (ACE).
(3) The composition for suppressing blood pressure elevation according to (1) above, wherein the composition suppresses blood pressure elevation by promoting nitric oxide (NO) production from vascular endothelial cells.
(4) The composition for suppressing blood pressure elevation according to any one of (1) to (3) above, wherein the composition for suppressing blood pressure elevation is a food or beverage.
(5) The composition for suppressing blood pressure elevation according to any one of (1) to (4) above, wherein the composition for suppressing blood pressure elevation is a beverage.
(6) The composition for suppressing blood pressure elevation according to (5) above, wherein the amount of xanthohumol in the beverage is 30 ppm by mass or more and less than 200 ppm by mass.
(7) The composition for suppressing blood pressure elevation according to (5) or (6) above, wherein the beverage is a tea-based beverage, a coffee beverage, an alcoholic beverage, a non-alcoholic beer-taste beverage, a carbonated beverage, a functional beverage, a fruit and/or vegetable-based beverage, a lactic beverage, a soy milk beverage, or flavored water.
(8) The composition for suppressing blood pressure elevation according to any one of (1) to (7) above, wherein the composition is labeled with one or both of the following function claims: "suppressing blood pressure elevation" and "for those with high blood pressure".

(9) A method for suppressing blood pressure elevation, the method including: administering or feeding xanthohumol to a subject.

(10) Use of xanthohumol for suppressing blood pressure elevation.

- Advantageous Effects of Invention

The present invention can provide a novel composition for suppressing blood pressure elevation, the composition being capable of suppressing blood pressure elevation by inhibiting angiotensin-converting enzyme (ACE) and thus promoting nitric oxide (NO) production from vascular endothelial cells.

DESCRIPTION OF EMBODIMENTS

The following describes details of the composition for suppressing blood pressure elevation, method for suppressing blood pressure elevation, and the like of the present invention.

In the present invention, the term "suppressing blood pressure elevation" is a concept that includes both suppression of blood pressure elevation and lowering of blood pressure.

The composition for suppressing blood pressure elevation of the present invention contains xanthohumol as an active ingredient.

As a result of intensive studies on components having an antihypertensive effect, the present inventors discovered that the xanthohumol has an angiotensin-converting enzyme (ACE) inhibitory effect and an effect of promoting nitric oxide (NO) production from vascular endothelial cells, and thus achieved the present invention.

Angiotensin-converting enzyme (ACE) present in vascular endothelial cells and elsewhere is an enzyme that converts inactive angiotensin I to active angiotensin II, and angiotensin II elevates blood pressure. Thus, the ACE inhibitory effect can suppress blood pressure elevation.

Vascular endothelial cells are cells that make up the lining of blood vessels, and are known to produce physiologically active substances such as nitric oxide (NO). Nitric oxide is one of vascular endothelium-derived relaxing factors. Promoting nitric oxide production from vascular endothelial cells can relax blood vessels and thus can suppress blood pressure elevation.

Xanthohumol has the ACE inhibitory effect and the effect of promoting nitric oxide (NO) production from vascular endothelial cells, and thus has an excellent antihypertensive effect.

The composition for suppressing blood pressure elevation of the present invention is suitably used to suppress blood pressure elevation by inhibiting angiotensin-converting enzyme (ACE). The composition for suppressing blood pressure elevation of the present invention is also suitably used to suppress blood pressure elevation by promoting nitric oxide (NO) production from vascular endothelial cells.

The xanthohumol is a component of hops that are an edible plant, and is suitable for foods and beverages. Xanthohumol can be extracted from hops with a solvent. For example, dried hops are ground into pellets which are then immersed in an organic solvent such as an alcohol for extraction. Then, the resulting liquid extract is concentrated and dried, followed by separation and purification by chromatography or the like, whereby xanthohumol can be obtained.

The temperature of extraction from hops, fractionation, and purification is preferably lower than 80°C, more preferably 5°C to 70°C, for example. Xanthohumol is commercially available, and such a commercial product can also be used. In the present invention, an ingredient derived from plants rich in xanthohumol may be used as long as the effect of the present invention can be achieved.

The composition for suppressing blood pressure elevation of the present invention is applicable for either therapeutic use (medical use) or non-therapeutic use (non-medical use). The "non-therapeutic" is a concept that does not include medical activities, i.e., a concept that does not include methods of surgery, therapy or diagnosis of humans.

The composition for suppressing blood pressure elevation of the present invention can be provided as a food or beverage, a pharmaceutical or quasi-pharmaceutical product, feed, or the like. The composition for suppressing blood pressure elevation of the present invention may be a food or beverage, a pharmaceutical or quasi-pharmaceutical product, feed, or the like by itself for suppressing blood pressure elevation; or may be a material, a preparation, or the like to be added to such a product.

Preferably, the composition for suppressing blood pressure elevation of the present invention is a composition for oral intake in order to sufficiently obtain the effect of the present invention. The composition for oral intake may be a food or beverage, a pharmaceutical or quasi-pharmaceutical product for oral administration, or feed, preferably a food or beverage or a pharmaceutical product for oral administration, more preferably a food or beverage.

The composition for suppressing blood pressure elevation of the present invention can contain optional additives and optional components, in addition to the active ingredient (xanthohumol) used in the present invention, as long as the effect of the present invention is not impaired. Such additives and components can be selected according to the composition form or the like. Generally, those that can be used in foods, beverages, pharmaceutical products, quasi-pharmaceutical products, feed, and the like can be used.

When the composition for suppressing blood pressure elevation of the present invention is provided as a food or beverage, a pharmaceutical or quasi-pharmaceutical product, feed, or the like, any common method can be used for the production. For example, the composition for suppressing blood pressure elevation which contains xanthohumol can also be produced by adding a hop extract in the production.

For example, when the composition for suppressing blood pressure elevation of the present invention is provided as a food or beverage, various types of foods or beverages can be provided by, for example, adding a component usable for foods and beverages (e.g., a food material or an optional food additive) to the active ingredient used in the present invention. Non-limiting examples of the food or beverage include general foods and beverages, health foods, health beverages, foods with function claims, foods for specified health uses, and foods and beverages for the sick. The health foods, foods with function claims, foods for specified health uses, and the like can be used in various forms of preparations such as fine granules, tablets, granules, powders, capsules, chewable tablets, syrups, liquids, and liquid foods.

An example of a preferred embodiment of the composition for suppressing blood pressure elevation of the present invention may be a beverage.

Preferably, the beverage in the present invention is a tea-based beverage, a coffee beverage, an alcoholic beverage, a non-alcoholic beer-taste beverage, a carbonated beverage, a functional beverage, a fruit and/or vegetable-based beverage, a lactic beverage, a soy milk beverage, or flavored water.

When the beverage in the present invention is a tea-based beverage, preferably, it is a black tea beverage or a sugarless tea beverage. Examples of the sugarless tea beverages include green tea beverages, oolong tea beverages, barley tea beverages, brown rice tea beverages, adlay tea beverages, and sugarless black tea beverages.

When the beverage in the present invention is a coffee beverage, preferably, it is a packaged coffee beverage or liquid coffee.

Examples of the alcoholic beverage include beer, beer-based beverages, and alcoholic beverages other than the beer and beer-based beverages.

When the beverage in the present invention is a beer-based beverage, preferably, it is low-malt beer or a beer-like beverage.

When the beverage in the present invention is an alcoholic beverage other than the beer and beer-based beverages, preferably, it is *shochu,* a *shochu* highball, a liqueur, cocktail, a spirit, or a whisky.

The term "non-alcoholic beer-taste beverage" as used herein refers to carbonated beverages with beer-like flavors, which are non-fermented non-alcoholic beverages, substantially free of alcohols. Here, the non-alcoholic beer-taste beverage does not exclude beverages containing a trace amount (undetectable amount) of alcohol.

When the beverage in the present invention is a carbonated beverage, preferably, it is a cola-flavored beverage, a clear carbonated beverage, ginger ale, a fruit juice-based carbonated beverage, a milk-containing carbonated beverage, or a sugarless carbonated beverage.

When the beverage in the present invention is a functional beverage, preferably, it is a sports drink, an energy drink, a health-supporting beverage, or a jelly drink pouch.

When the beverage in the present invention is a fruit and/or vegetable-based beverage, preferably, it is a 100% fruit juice, a fruit-containing beverage, a soft drink with a low fruit juice content, a pulp-containing fruit juice, or a pulp-containing beverage.

When the beverage in the present invention is a lactic beverage, preferably, it is milk, a yogurt drink, a lactic acid bacteria beverage, or a milk-containing soft drink.

When the beverage in the present invention is a soy milk beverage, preferably, it is soy milk or a soybean beverage.

The form of the beverage in the present invention is not particularly limited. Examples include packaged beverages. Packages for the packaged beverages are not particularly limited. Packages in any form and of any material may be used. For example, any of the following commonly used packages can be used: metal packages such as aluminum cans and steel cans; resin containers such as plastic bottles; paper containers such as cartons; glass containers such as glass bottles; and wooden containers such as barrels. The beverage is filled and sealed in any of these packages, whereby a packaged beverage can be obtained.

For example, the beverage in the present invention can be prepared by adding xanthohumol to a substance that is used in the beverage production (e.g., any food ingredient or food additive).

When the composition for suppressing blood pressure elevation of the present invention is provided as a pharmaceutical or quasi-pharmaceutical product, various dosage forms of pharmaceutical or quasi-pharmaceutical products can be provided by, for example, adding a pharmacologically acceptable carrier, an optional additive, or the like to the active ingredient (xanthohumol) used in the present invention. Such a carrier, an additive, or the like may be of any pharmacologically acceptable type that can be used in pharmaceutical or quasi-pharmaceutical products. Examples thereof include excipients, binders, disintegrants, lubricants, antioxidants, and colorants. One or more of these can be used. The form of administration (intake) of the pharmaceutical or quasi-pharmaceutical product may be oral, enteral, transmucosal, or administration, injection, or the like. Oral administration is preferred in order to more sufficiently obtain the effect of the present invention.

When the composition for suppressing blood pressure elevation of the present invention is provided as a pharmaceutical product, preferably, it is a pharmaceutical product for oral administration. Examples of dosage forms of preparations for oral administration include liquids, tablets, powders, fine granules, granules, sugar-coated tablets, capsules, suspensions, emulsions, and chewable tablets. The pharmaceutical product may be a pharmaceutical product for non-human animals.

Providing the composition for suppressing blood pressure elevation of the present invention as feed only requires adding the active ingredient (xanthohumol) used in the present invention to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of xanthohumol in the composition for suppressing blood pressure elevation of the present invention is not limited, and may be suitably set according to the composition form. For example, the amount of xanthohumol in the composition is preferably 0.0001 wt% or more, more preferably 0.0003 wt% or more, and is preferably 90 wt% or less. In one embodiment, the amount of xanthohumol in the composition for suppressing blood pressure elevation may be 0.0001 to 90 wt%. In one embodiment, when the composition for suppressing blood pressure elevation of the present invention is provided as a food or beverage, a pharmaceutical or quasi-pharmaceutical product, feed, or the like, preferably, the amount of xanthohumol is in the above ranges.

For example, when the composition for suppressing blood pressure elevation of the present invention is provided as a beverage, the amount of xanthohumol is preferably 1 ppm by mass or more or more preferably 3 ppm by mass or more.

When the amount of xanthohumol is 1 ppm by mass or more, the NO production from vascular endothelial cells is promoted, which results in an antihypertensive effect. When the amount of xanthohumol is 3 ppm by mass or more, the ACE inhibitory effect of xanthohumol is obtained, and the antihypertensive effect can be more sufficiently obtained.

When the composition for suppressing blood pressure elevation of the present invention is provided as a beverage, the amount of xanthohumol is more preferably 10 ppm by mass or more, still more preferably 30 ppm by mass or more.

A larger amount of xanthohumol is likely to result in a higher antihypertensive effect, but increases the bitterness derived from xanthohumol. For example, when the composition for suppressing blood pressure elevation is provided as a beverage, the amount of xanthohumol in the composition for suppressing blood pressure elevation of the present invention is preferably less than 200 ppm by mass, in order to provide aroma that makes the beverage palatable for people to consume.

When the composition for suppressing blood pressure elevation of the present invention is provided as a beverage, the amount of xanthohumol is preferably less than 200 ppm by mass, more preferably 175 ppm by mass or less.

When the composition for suppressing blood pressure elevation of the present invention is provided as a beverage, the amount of xanthohumol is preferably 3 ppm by mass or more and less than 200 ppm by mass, more preferably 10 ppm by mass or more and less than 200 ppm by mass, still more preferably 30 ppm by mass or more and less than 200 ppm by mass. The amount is preferably 3 ppm by mass or more and 175 ppm by mass or less, more preferably 10 ppm by mass or more and 175 ppm by mass or less, still more preferably 30 ppm by mass or more and 175 ppm by mass or less.

The amount of xanthohumol in the composition for suppressing blood pressure elevation of the present invention can be quantified by, for example, high-performance liquid chromatography (HPLC) or using an LC-MS/MS system. Advantageously, xanthohumol is easy to quantify and thus easy to use as an active ingredient of foods with function claims and the like which require quantitative analysis and standardization of active ingredients.

The intake (dosage) of the composition for suppressing blood pressure elevation of the present invention is not limited, as long as it is the amount (effective amount) that provides the antihypertensive effect. The intake may be suitably set according to the dosage form and administration method.

In one embodiment, when the composition for suppressing blood pressure elevation of the present invention is orally fed or administered to a human (adult), the intake of the composition in terms of xanthohumol is preferably 1 to 200 mg, more preferably 5 to 60 mg, per 60 kg per day. Feeding or administration of xanthohumol in the above amount in one or more portions per day, for example, one to several portions (e.g., two to three portions) per day, is preferred.

Preferably, the composition for suppressing blood pressure elevation of the present invention is fed or administered by a method appropriate to the form. Preferably, the composition for suppressing blood pressure elevation of the present invention is orally fed (orally administered) in order to sufficiently obtain the effect of the present invention.

A subject to be fed (administered) with the composition for suppressing blood pressure elevation of the present invention is not limited. The subject is preferably a human or non-human mammal, more preferably a human.

Preferably, the subject to be fed or administered with the composition for suppressing blood pressure elevation of the present invention is one who needs or wants to suppress blood pressure elevation. Examples of the subject include humans with hypertension (grade I hypertension, grade II hypertension, and grade III hypertension based on the "Guidelines for the Management of Hypertension" stipulated by the Japanese Society of Hypertension) and humans with high-normal blood pressure (based on the same guidelines). The composition for suppressing blood pressure elevation of the present invention can also be used on a healthy person in order to, for example, prevent a condition or disease that is likely to be prevented or ameliorated by suppression of blood pressure elevation.

The composition for suppressing blood pressure elevation of the present invention may be labeled with function claims based on the antihypertensive effect. For example, the composition for suppressing blood pressure elevation of the present invention may be labeled with one or both of the following function claims: "suppressing blood pressure elevation" and "for those with high blood pressure".

In one embodiment of the present invention, preferably, the composition for suppressing blood pressure elevation of the present invention is a food or beverage labeled with one or both of the above function claims. The labels may be labels indicating use for obtaining these functions.

The present invention also encompasses the following method:

a method for suppressing blood pressure elevation, the method including administering or feeding xanthohumol to a subject.

Preferably, the method for suppressing blood pressure elevation is a method for suppressing blood pressure elevation by inhibiting angiotensin-converting enzyme (ACE) and thus promoting nitric oxide (NO) production from vascular endothelial cells.

The method may be a therapeutic or non-therapeutic method.

Administering or feeding xanthohumol to a subject is likely to result in an ACE inhibitory effect and an effect of promoting nitric oxide (NO) production from vascular endothelial cells. These effects make it possible to control blood pressure elevation.

The present invention also encompasses the following use:

use of xanthohumol for suppressing blood pressure elevation.

Preferably, the use of xanthohumol is use of xanthohumol for suppressing blood pressure elevation by an angiotensin-converting enzyme (ACE) inhibitory effect and an effect of promoting nitric oxide (NO) production from vascular endothelial cells.

Preferably, the use is for a human or non-human mammal, more preferably a human. The use may be therapeutic or non-therapeutic use.

Preferred embodiments of the method and the use are as described above for the composition for suppressing blood pressure elevation of the present invention.

In the method and the use, preferably, xanthohumol is administered or fed in one or more portions per day, for example, one to several portions (e.g., two to three portions) per day, to a subject.

The method and the use only require administering or feeding xanthohumol in an amount (effective amount) that provides the antihypertensive effect to a subject. A preferred intake of xanthohumol, a preferred subject to be administered, a preferred administration method, and the like are as described above for the composition for suppressing blood pressure elevation of the present invention described above. Xanthohumol may be directly administered or fed, or may be administered or fed as a composition containing xanthohumol. For example, the composition for suppressing blood pressure elevation of the present invention may be administered as a pharmaceutical product or may be fed as a food or beverage.

Xanthohumol can also be used to produce a food or beverage, a pharmaceutical or quasi-pharmaceutical product, feed, or the like that is used to suppress blood pressure elevation. In one embodiment, the present invention also encompasses use of xanthohumol for producing a composition for suppressing blood pressure elevation; and use of xanthohumol for producing a composition for improving blood pressure.

EXAMPLES

The following provides examples that more specifically describe the present invention. The present invention is not limited to these examples.

<Examples 1 to 3>

Angiotensin-converting enzyme (ACE) inhibitory effect of xanthohumol

The angiotensin-converting enzyme (ACE) inhibitory effect of xanthohumol was evaluated by the following procedure.

Specifically, the ACE inhibitory activity was measured for the case where xanthohumol (produced by Gifu Shellac Manufacturing Co., Ltd.) was added as a test substance (n = 4 per level), using an ACE Kit-WST (Dojindo Laboratories) according to the instructions accompanying the kit. Table 1 below shows the xanthohumol concentration of test substance-added groups.

A level with addition of distilled water instead of xanthohumol was set as a control group.

A level with no addition of xanthohumol which was measured in an ACE (enzyme)-free reaction system was set as an enzyme-free group.

The ACE inhibitory activity (%) of the test substance was calculated using the following formula 1. Significant difference was tested between the groups (control group vs. test substance-added group) by Student's t-test (significant level: $p < 0.05$).

```
(Formula 1)
ACE inhibitory activity (%)
= 100 × ((absorbance at 450 nm of control group) -
(absorbance at 450 nm in test substance-added
group))/((absorbance at 450 nm of control group) -
(absorbance at 450 nm of enzyme-free group))
```

[Table 1]

|  | Xanthohumol concentration ($\mu$g/mL) | ACE inhibitory activity (%) | |
| --- | --- | --- | --- |
|  |  | Average | Standard error |
| Example 1 | 3 | 3.33 | 1.17 |
| Example 2 | 10 | 15.79 | 0.43 |
| Example 3 | 30 | 19.51 | 0.33 |

According to the above results, all the levels in Examples 1 to 3 showed significant differences relative to the control group, confirming the ACE inhibitory effect of xanthohumol. Xanthohumol is likely to exhibit an antihypertensive effect based on the ACE inhibitory effect.

The unit (pg/mL) in the table is equivalent to ppm.

<Examples 4 to 6>

Effect of xanthohumol in promoting nitric oxide (NO) production from vascular endothelial cells

The effect of xanthohumol (produced by Gifu Shellac Manufacturing Co., Ltd.) in promoting nitric oxide (NO) production from vascular endothelial cells was evaluated by the following procedure.

Specifically, human umbilical vein endothelial cells (HUVECs, Lonza) were treated with xanthohumol for 24 hours, and the amount of nitric oxide (NO) production was measured (n = 5).

(Procedure)

HUVECs in a medium (EGM-2, Lonza) were seeded onto a 96-well plate at 10000 cells/0.1 mL/well, and cultured in a $CO_2$ incubator. On the following day, the medium of a xanthohumol-added group was replaced by a medium to which xanthohumol was added; the control group was transferred to a control medium; and the positive control group was transferred to a medium containing positive control (Lovastatin, 10 $\mu$M). Then, these groups were cultured in a $CO_2$ incubator for additional 24 hours.

The medium to which xanthohumol was added was prepared by dissolving xanthohumol in dimethyl sulfoxide (DMSO) and adding the solution to a medium such that the final concentration of xanthohumol was 1 $\mu$g/mL, 3 $\mu$g/mL, or 10 $\mu$g/mL. The control medium was prepared by adding DMSO to a medium to the same concentration as that in the xanthohumol-added group. The medium containing positive control was prepared by adding a solution of lovastatin in DMSO to a medium such that the final concentration of lovastatin (produced by FUJIFILM Wako Pure Chemical Corporation) was 10 $\mu$M.

After culturing for 24 hours, diaminofluorescein-2 diacetate (DAF-2, Goryo Chemical, Inc.) was added to each medium to a final concentration of 10 $\mu$M, followed by incubation in a $CO_2$ incubator for two hours. Subsequently, the culture supernatant was transferred to a 96-well plate (black plate), and the fluorescence intensity (ex. 495 nm, em. 515 nm) was measured. The fluorescence intensity relative to the fluorescence intensity of the control group was determined as the relative amount of NO released. Significant difference was tested between the groups (control group vs. xanthohumol-added group) by Student's t-test (significant level: $p < 0.05$). Table 2 below shows the relative amount of NO released (%) of each group (average $\pm$ standard error).

[Table 2]

|  | Xanthohumol concentration ($\mu$g/mL) | Relative amount of NO released (%) | |
|---|---|---|---|
|  |  | Average | Standard deviation |
| Comparative Example 1 | 0 | 100.0 | 1.8 |
| Example 4 | 1 | 108.3 | 2.5 |
| Example 5 | 3 | 124.8 | 3.6 |
| Example 6 | 10 | 147.5 | 7.9 |

All the xanthohumol-added groups were significantly different from the control group. The relative amount of NO released of the positive control group was $122.8 \pm 2.7\%$ (significantly different). The above results show that xanthohumol has a physiological effect of increasing nitric oxide (NO) production from vascular endothelial cells (vascular endothelial function improving effect).

<Test Examples 1 to 8>

Preparation of xanthohumol-containing beverage

A xanthohumol emulsion having a composition shown in Table 3 below was prepared, and the xanthohumol emulsion was added to water, whereby xanthohumol-containing beverages (Test Examples 1 to 8) were prepared. Table 4 shows the xanthohumol concentration (ppm) in each xanthohumol-containing beverage. Xantho-Flav (produced by Hopsteiner) was used as a xanthohumol-containing material. A beverage having a composition shown in Table 3 below with no addition of xanthohumol (Test Example 1) was set as the control.

[Table 3]

|  | Proportion (mass%) |
|---|---|
| Xanthohumol-containing material | 7.5 |
| Emulsifier | 73.5 |
| Propylene glycol | 9 |
| Oil and/or fat | 5 |
| Water | 5 |

[Table 4]

|  | Xanthohumol concentration (ppm) |
|---|---|
| Test Example 1 | 0 |
| Test Example 2 | 50 |
| Test Example 3 | 100 |
| Test Example 4 | 150 |
| Test Example 5 | 175 |
| Test Example 6 | 200 |

(continued)

| | Xanthohumol concentration (ppm) |
|---|---|
| Test Example 7 | 250 |
| Test Example 8 | 300 |

<Evaluation of aroma in xanthohumol-containing beverage>

The resulting xanthohumol-containing beverages (Test Examples 1 to 8) were subjected to sensory evaluation by three panelists. Each beverage was individually evaluated based on the following four-point scale evaluation criteria and point allocation method, and an average score of the three panelists was calculated after completion of the evaluation. Table 5 below shows the results.

<Evaluation criteria and point allocation method>

No bitterness was sensed: 0 points
Slight bitterness was sensed but acceptable: 1 point
Bitterness was sensed but acceptable: 2 points
The bitterness was strong and unacceptable: 3 points

[Table 5]

| | Score (points) | |
|---|---|---|
| | Average | Standard deviation |
| Test Example 1 | 0.0 | 0.0 |
| Test Example 2 | 0.7 | 0.6 |
| Test Example 3 | 1.3 | 0.6 |
| Test Example 4 | 2.0 | 0.0 |
| Test Example 5 | 2.0 | 0.0 |
| Test Example 6 | 3.0 | 0.0 |
| Test Example 7 | 3.0 | 0.0 |
| Test Example 8 | 3.0 | 0.0 |

The above results show that when the xanthohumol concentration in the beverage was 200 ppm by mass or more, the bitterness was strong and unacceptable. This confirmed that the unacceptable bitterness was reduced when the xanthohumol concentration in the beverage was less than 200 ppm by mass, and particularly that the bitterness, even if tasted, was considered to be acceptable when the xanthohumol concentration in the beverage was 175 ppm by mass or less. Thus, xanthohumol can be added to a beverage without sacrificing the original taste of the drink when the xanthohumol concentration in the beverage is less than 200 ppm by mass, and such a beverage is palatable to drink. In particular, xanthohumol can be added to a beverage without sacrificing the original taste of the drink when the xanthohumol concentration in the beverage is 175 ppm by mass or less, and such a beverage is palatable to drink.

In other words, when the composition for suppressing blood pressure elevation of the present invention is a beverage, the beverage is suitable for regular and continuous consumption, and the antihypertensive effect based on the angiotensin-converting enzyme (ACE) inhibitory effect and the effect of promoting NO production from vascular endothelial cells can be effectively obtained.

**Claims**

**1.** A composition for suppressing blood pressure elevation, the composition comprising:

xanthohumol as an active ingredient.

2. The composition for suppressing blood pressure elevation according to claim 1,
   wherein the composition suppresses blood pressure elevation by inhibiting angiotensin-converting enzyme (ACE).

3. The composition for suppressing blood pressure elevation according to claim 1,
   wherein the composition suppresses blood pressure elevation by promoting nitric oxide (NO) production from vascular endothelial cells.

4. The composition for suppressing blood pressure elevation according to any one of claims 1 to 3,
   wherein the composition is a food or beverage.

5. The composition for suppressing blood pressure elevation according to any one of claims 1 to 4,
   wherein the composition is a beverage.

6. The composition for suppressing blood pressure elevation according to claim 5,
   wherein the amount of xanthohumol in the beverage is 30 ppm by mass or more and less than 200 ppm by mass.

7. The composition for suppressing blood pressure elevation according to claim 5 or 6,
   wherein the beverage is a tea-based beverage, a coffee beverage, an alcoholic beverage, a non-alcoholic beer-taste beverage, a carbonated beverage, a functional beverage, a fruit and/or vegetable-based beverage, a lactic beverage, a soy milk beverage, or flavored water.

8. The composition for suppressing blood pressure elevation according to any one of claims 1 to 7,
   wherein the composition is labeled with one or both of the following function claims: "suppressing blood pressure elevation" and "for those with high blood pressure".

9. A method for suppressing blood pressure elevation, the method comprising:
   administering or feeding xanthohumol to a subject.

10. Use of xanthohumol for suppressing blood pressure elevation.

## INTERNATIONAL SEARCH REPORT

| International application No. |
|---|
| PCT/JP2019/046983 |

**A. CLASSIFICATION OF SUBJECT MATTER**
A61K 31/121(2006.01)i; A61P 9/12(2006.01)i; A61P 43/00(2006.01)i; A23L 33/105(2016.01)i; A23L 2/52(2006.01)i
FI: A23L33/105; A61K31/121; A61P9/12; A61P43/00 111; A23L2/00 F
According to International Patent Classification (IPC) or to both national classification and IPC

**B. FIELDS SEARCHED**

Minimum documentation searched (classification system followed by classification symbols)
A61K31/121; A61P9/12; A61P43/00; A23L33/105; A23L2/52

| Documentation searched other than minimum documentation to the extent that such documents are included in the fields searched | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
CAplus/MEDLINE/EMBASE/BIOSIS/WPIDS/FSTA (STN), JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2005-13150 A (ASAHI BREWERIES, LTD.) 20.01.2005 (2005-01-20) claims, paragraphs [0041], [0047], [0048], [0068], [0069], examples | 1-10 |
| X | JP 2015-40839 A (POLA CHEMICAL INDUSTRIES, INC.) 02.03.2015 (2015-03-02) paragraphs [0061]-[0063] | 1-10 |
| X | KR 10-2009-0084439 A (PUKYONG NATIONAL UNIVERSITY INDUSTRY UNIVERSITYCOOPERATION FOUNDATION) 05.08.2009 (2009-08-05) claims, examples, paragraphs [0080], [0100]-[0104] | 1-10 |
| X | JP 2013-43850 A (LION CORP.) 04.03.2013 (2013-03-04) claims, examples, paragraphs [0030], [0034] | 1-10 |
| X | US 2010/0160450 A1 (KUHRTS, Eric) 24.06.2010 (2010-06-24) paragraphs [0011]-[0014], [0036] | 1-10 |

☒ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 19 February 2020 (19.02.2020) | 03 March 2020 (03.03.2020) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
|---|
| PCT/JP2019/046983 |

C (Continuation). DOCUMENTS CONSIDERED TO BE RELEVANT

| Category* | Citation of document, with indication, where appropriate, of the relevant passages | Relevant to claim No. |
|---|---|---|
| A | US 2016/0220542 A1 (UNIVERSITAETSKLINIKUM FREIBURG) 04.08.2016 (2016-08-04) paragraphs [0066]-[0075] | 1-10 |
| A | ZHANG, Nenling et al., "A new formylated chalcone from Humulus lupulus with protective effect on HUVECs injury by angiotensin II", Natural Product Research, 24 November 2017, vol. 33, no. 5, pp. 617-621 abstract, page 620, paragraph [0003], fig. 2 | 1-10 |
| A | CN 105963284 A (SHANGHAI UNIVERSITY OF TRADITIONAL CHINESE MEDICINE) 28.09.2016 (2016-09-28) claims, examples 3-5 | 1-10 |
| A | CN 105982881 A (SHANGHAI UNIVERSITY OF TRADITIONAL CHINESE MEDICINE) 05.10.2016 (2016-10-05) claims, examples | 1-10 |
| A | JP 2006-306800 A (KIRIN BREWERY CO., LTD.) 09.11.2006 (2006-11-09) claims | 1-10 |
| A | JP 2011-256133 A (NATIONAL UNIVERSITY CORPORATION HOKKAIDO UNIVERSITY, SAPPORO BREWERIES LTD.) 22.12.2011 (2011-12-22) paragraphs [0054]-[0055], claims | 1-10 |
| A | JP 2009-502973 A (BIOACTIVES, INC.) 29.01.2009 (2009-01-29) claims | 1-10 |
| A | JP 2009-269927 A (TAKARA BIO INC.) 19.11.2009 (2009-11-19) claims, paragraphs [0025], [0070] | 1-10 |

Form PCT/ISA/210 (continuation of second sheet) (January 2015)

INTERNATIONAL SEARCH REPORT
Information on patent family members

International application No.

PCT/JP2019/046983

| Patent Documents referred in the Report | Publication Date | Patent Family | Publication Date |
|---|---|---|---|
| JP 2005-13150 A | 20 Jan. 2005 | (Family: none) | |
| JP 2015-40839 A | 02 Mar. 2005 | (Family: none) | |
| KR 10-2009-0084439 A | 05 Aug. 2009 | (Family: none) | |
| JP 2013-43850 A | 04 Mar. 2013 | (Family: none) | |
| US 2010/0160450 A1 | 24 Jun. 2010 | WO 2008/095122 A2 KR 10-2009-0114427 A | |
| US 2016/0220542 A1 | 04 Aug. 2016 | WO 2012/022659 A2 EP 2422777 A1 | |
| CN 105963284 A | 28 Sep. 2016 | (Family: none) | |
| CN 105982881 A | 05 Oct. 2016 | (Family: none) | |
| JP 2006-306800 A | 09 Nov. 2006 | (Family: none) | |
| JP 2011-256133 A | 22 Dec. 2011 | (Family: none) | |
| JP 2009-502973 A | 29 Jan. 2009 | US 2009/0209654 A1 claims WO 2007/016578 A2 EP 1909584 A1 KR 10-2008-0063748 A CN 101272689 A | |
| JP 2009-269927 A | 19 Nov. 2009 | US 2004/0002423 A1 claims, paragraphs [0026], [0109] WO 2001/076614 A1 EP 1277473 A1 CN 1422159 A KR 10-2003-0013382 A | |

Form PCT/ISA/210 (patent family annex) (January 2015)

## REFERENCES CITED IN THE DESCRIPTION

**Patent documents cited in the description**

- JP 2002345433 A
- JP 2003310240 A
- JP 2012153659 A

**Non-patent literature cited in the description**

- *J Biochem.,* August 2008, vol. 94 (2), 619-22
- *PloS.ONE,* vol. 7 (11), e49493
- *J Nutr Sci Vitaminol,* vol. 54, 95-98